# EUROPEAN PATENT APPLICATION

(11) **EP 4 509 501 A1**
(43) Date of publication of application: **19.02.2025**
(21) Application number: 23787709.7
(22) Date of filing: 11.04.2023
(51) Int. Cl.: C07D 403/04, A61P 35/00, A61P 31/14, A61P 31/16, A61P 31/12, A61P 11/00, A61K 31/4164

(54) **DEUTERATED HETEROCYCLIC KETONE COMPOUND AND USE THEREOF**

(30) Priority: 12.04.2022 CN 202210379884
(71) Applicant: Hinova Pharmaceuticals Inc., Chengdu, Sichuan 610041 (CN)
(72) Inventor: DU, Wu, Chengdu, Sichuan 610041 (CN); LI, Xinghai, Chengdu, Sichuan 610041 (CN); CHEN, Song, Chengdu, Sichuan 610041 (CN)
(74) Representative: Altmann Stößel Dick Patentanwälte PartG mbB
(86) International application number: PCT/CN2023/087636
(87) International publication number: WO 2023/198066

(57) **Abstract**

The present invention relates to a compound represented by formula (I) or an optical isomer, a pharmaceutically acceptable salt, a hydrate or a solvate thereof, wherein R¹-R¹⁷ are each independently selected from hydrogen and deuterium, R¹⁸ and R¹⁹ are hydrogen, and R¹-R¹⁹ are not simultaneously hydrogen. The deuterated compound and the salt, hydrate or solvate thereof have excellent metabolic stability and pharmacokinetic properties, thus having excellent application prospects in the manufacture of tubulin inhibitors, anti-cancer drugs, antiviral drugs, and drugs for the treatment of novel coronavirus pneumonia.

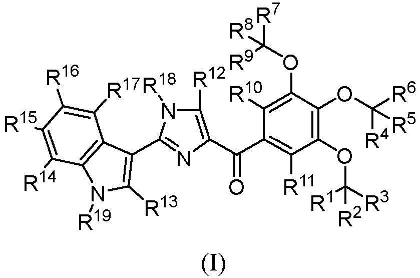

## Description

### Technical field

The present invention relates to a deuterated heterocyclic ketone compound and uses thereof.

### Background

Sabizabulin (VERU-111, ABI-231) is an orally effective tubulin inhibitor, with the structure 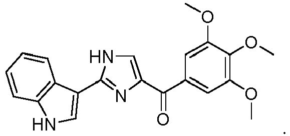 It has significant anti-proliferative activity against prostate cancer and other tumor cell lines, and is clinically used to treat advanced breast cancer and prostate cancer. It has been found that Sabizabulin has anti-inflammatory effect and can prevent cytokine storm caused by COVID-19 infection. In the phase III clinical trial, the death risk of patients was relatively reduced by 55%.

Deuterated drugs refer to the substitution of some hydrogens in drug molecules with deuterium. Due to the shape and volume of deuterium in drug molecules being close to those of hydrogen, deuterated drugs generally retain the biological activity and selectivity of the original drug. Since the stability of the C-D bond is better compared to the C-H bond, deuterated drugs are less likely to break the C-D bond during chemical reactions, resulting in an extended half-life.

Due to the complex metabolic processes of biological systems, the pharmacokinetic properties of medicaments in organisms are influenced by various factors and exhibit corresponding complexity. Compared with corresponding non-deuterated drugs, the changes in the pharmacokinetic properties of deuterated drugs exhibit obvious randomness and unpredictability. Deuterium substitution at certain sites may not only fail to prolong the half-life, but may also shorten it (Scott L. Harbeson, Roger D. Tung. Deuterium in Drug Discovery and Development, P405-406.), affecting its pharmacokinetic properties; on the other hand, hydrogens at certain positions of drug molecules are not easily substituted with deuterium due to steric hindrance and other reasons. Therefore, deuteration of drugs is not arbitrary, and the deuterated sites are unpredictable.

If Sabizabulin can be deuterated, its pharmacokinetic properties can be further improved, and its side effects may be reduced, which is conducive to the further development of medicaments for the treatment of malignant tumors and novel coronavirus infection, that is of great significance.

### Content of the invention

The object of the present invention is to provide a deuterated Sabizabulinb compound.

The present invention provides a compound represented by formula (I) or an optical isomer, a pharmaceutically acceptable salt, a hydrate, or a solvate thereof: 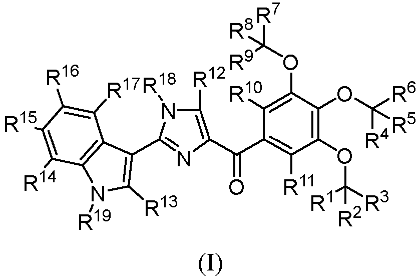 wherein R¹-R¹⁷ are each independently selected from hydrogen and deuterium, R¹⁸ and R¹⁹ are hydrogen, and R¹-R¹⁹ are not hydrogen at the same time.

Further, the compound has a structure as represented by formula (II):

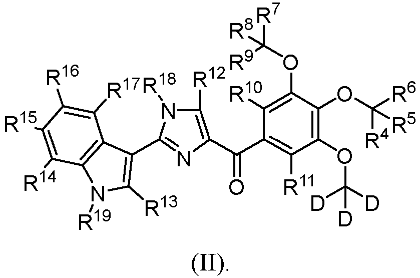

Further, the compound has a structure as represented by formula (III):

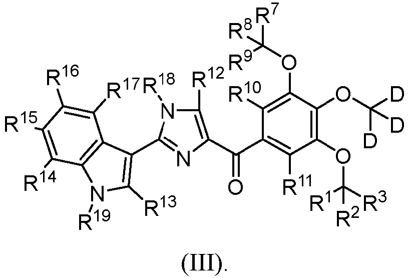

More further, the compound has a structure as represented by formula (IV):

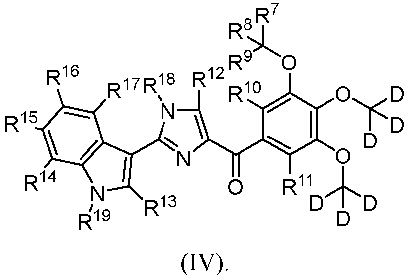

More further, the compound has a structure as represented by formula (V):

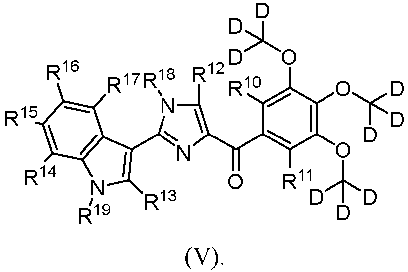

Further, the compound has a structure as represented by formula (VI):

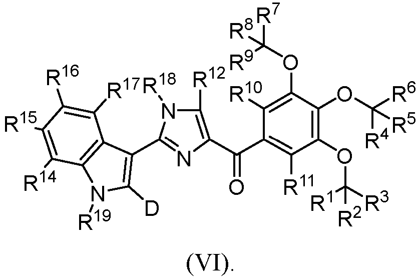

Further, the compound has a structure as represented by formula (VII):

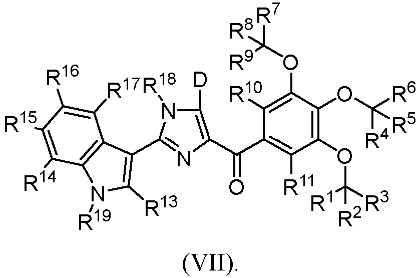

Further, the compound has a structure as represented by formula (VIII):

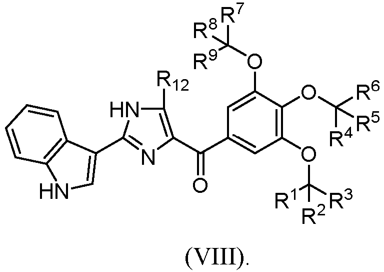

More further, in the above compounds, R₁, R₂, and R₃ are all hydrogen at the same time or are all deuterium at the same time; R₄, R₅, and R₆ are all hydrogen at the same time or are all deuterium at the same time; R₇, R₈, and R₉ are all hydrogen at the same time or are all deuterium at the same time.

Further, the compound is selected from the group consisting of:

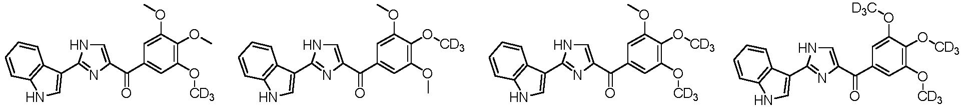

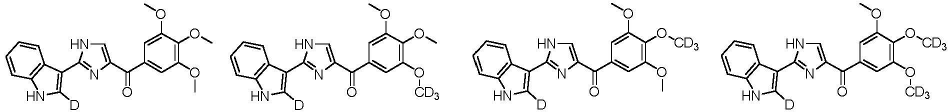

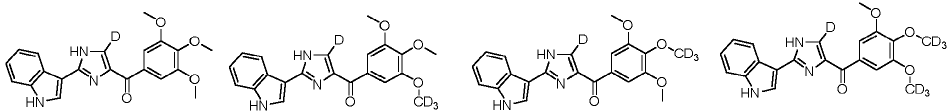

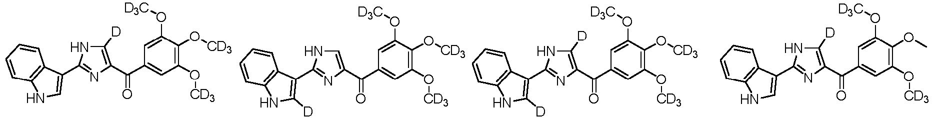

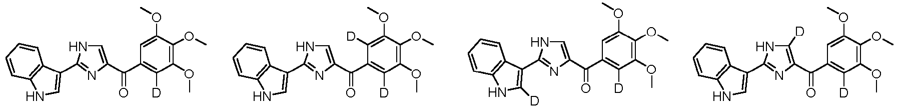

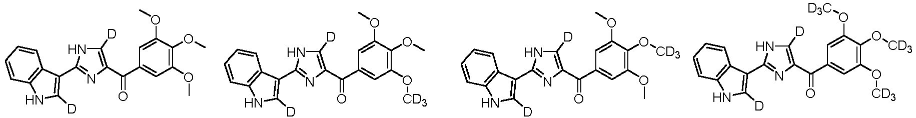

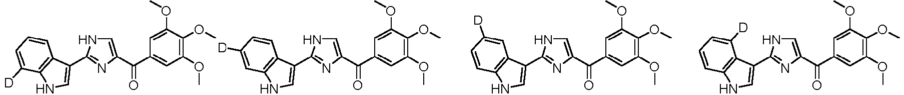

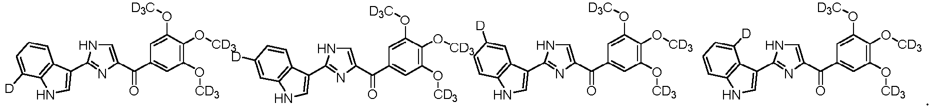

Further, the pharmaceutically acceptable salt includes phosphate, D-camphorsulfonate, hydrochloride, hydrobromate, hydrofluorate, sulfate, nitrate, formate, acetate, propionate, oxalate, malonate, succinate, fumarate, maleate, lactate, malate, tartrate, citrate, picrate, methanesulfonate, phenylmethanesulfonate, benzenesulfonate, aspartate or glutamate of the compound.

The present invention also provides the use of the above compound or an optical isomer, a pharmaceutically acceptable salt, a hydrate, or a solvate thereof in the manufacture of medicaments for the treatment of cancer, COVID-19 infection, influenza virus infection, syncytial virus (SV) infection and/or acute respiratory distress syndrome (ARDS).

Further, the cancer includes but is not limited to melanoma, prostate cancer or breast cancer.

The present invention also provides the use of the above compound or an optical isomer, a pharmaceutically acceptable salt, a hydrate, or a solvate thereof in the manufacture of tubulin inhibitors.

The present invention also provides a medicament, which is a preparation formed by the above compound or an optical isomer, a pharmaceutically acceptable salt, a hydrate, or a solvate thereof, as the active ingredient, in combination with pharmaceutically acceptable excipients.

The beneficial effects of the present invention: the deuterated compound obtained by deuteration of Sabizabulin has anti-cancer activity, as well as better metabolic stability and pharmacokinetics than Sabizabulin, possessing excellent application prospects.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Examples

The raw materials and equipment used in the present invention are known products obtained by purchasing those commercially available.

### Example 1: Synthesis of (2-(1H-indol-3-yl)-1H-imidazol-4-yl)(3,4,5-tri(methoxy-d₃)phenyl) ketone

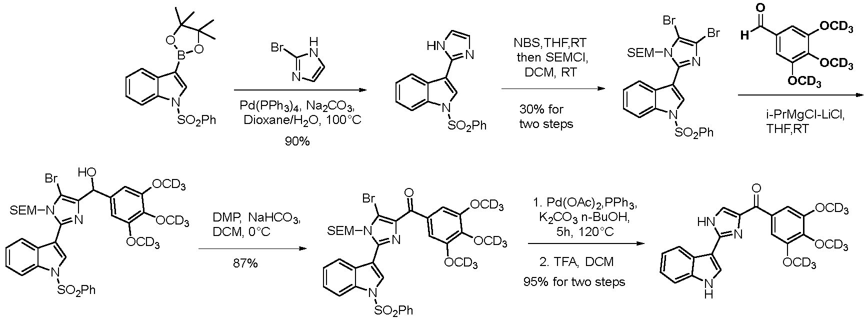

### Step 1: Synthesis of 3-(1H-imidazol-2-yl)-1-(phenylsulfonyl)-1H-indole

To a 100 mL round-bottom flask, were added compound 1-(phenylsulfonyl)-3-(4,4,5,5-tetramethyl-1,3,2-dioxybenzaldehyde-2-yl)-1H-indole (2 g, 5.22 mmol) and compound 2-bromo-1H-imidazole (1 eq., 0.77 g, 5.22 mmol), to which was added anhydrous dioxane/H₂O (30/10 ml), followed by successive addition of Na₂CO₃ (2 eq., 1.45 g, 10.44 mmol) and tetrakis(triphenylphosphine)palladium (0.1eq., 0.6 g, 0.52 mmol), and then the system was purged with argon for three times. The mixture was allowed to react overnight at 100 °C, and then saturated NH₄Cl solution was added to quench the reaction. The resultant solution was extracted with EA (5*50 mL). The organic phase was rotatory evaporated to dry, and the residue was purified by column chromatography (EA/PE = 1/1), to provide compound 3-(1H-imidazol-2-yl)-1-(phenylsulfonyl)-1H-indole (1.8 g, 5.52 mmol, >100%). LC/MS (ESI+) calcd for C₁₇H₁₃N₃O₂S ([M+H]⁺) *m*/*z* 324.07, found 324.2.

### Steps 2 and 3: Synthesis of 3-(4,5-dibromo-1-(2-(trimethylsilyl)ethoxy)methyl)-1H-imidazol-2-yl)-1-(phenylsulfonyl)-1H-indole

To a 100 mL round-bottom flask, were added compound 3-(1H-imidazol-2-yl)-1-(phenylsulfonyl)-1H-indole (1.8 g, 5.52 mmol) and 30 mL of anhydrous THF, to which was slowly added NBS (2.5 eq., 2.3g, 13.8 mmol) at room temperature. After stirring for 2h, saturated sodium thiosulfate solution was added to quench the reaction, and the resultant solution was extracted with EA for three times. The organic phase was rotatory evaporated to dry. To the residue, were added 30 mL of DCM and DIPEA (1.7 mL, 11.04 mmol), to which was slowly added SEMCl (1.2 eq., 1.2 mL, 6.8 mmol) in an ice water bath. The reaction was stirred for 2h, and then quenched by adding saturated NaHCO₃ solution. The resultant solution was extracted with DCM (4*30 mL), followed by purification over column chromatography (EA/PE = 1/5), to obtain compound 3-(4,5-dibromo-1-(2-(trimethylsilyl)ethoxy)methyl)-1H-imidazol-2-yl)-1-(phenylsulfonyl)-1H-indole (0.975 g, 1.60 mmol, 30%). LC/MS (ESI+) calcd for C₂₃H₂₅Br₂N₃O₃SSi ([M+H]⁺) *m*/*z* 611.97, 609.98, found 612.1, 614.1.

### Step 4. Synthesis of 3,4,5-tri(methoxy-d₃)benzaldehyde

In a 50 mL sealed tube, 3,4,5-trihydroxybenzaldehyde (2.0 g, 12.98 mmol) was weighed and added, to which were added 15 mL of DMF and K₂CO₃ (4.0 eq., 7.2 g, 51.94 mmol), followed by careful addition of CD₃I (4.0 eq., 3.5 mL, 51.94 mmol). The reaction was stirred overnight at 80 °C, and then cooled to room temperature, to which was added saturated NH₄Cl solution for quenching. The resultant solution was extracted with EA (5*50 mL). The organic phase was rotatory evaporated to dry, and the residue was purified by column chromatography (EA/PE = 1/3), to provide compound 3,4,5-tri(methoxy-*d*₃)benzaldehyde (2.15 g, 10.48 mmol, 81%).

### Step 5. (5-bromo-2-(1-(phenylsulfonyl)-1H-indol-3-yl)-1-(2-(trimethylsilyl)ethoxy) methyl)-1H-imidazol-4-yl)(3,4,5-tri(methoxy-d₃)phenyl)methanol

To a 100 mL round-bottom flask, were added 3-(4,5-dibromo-1-(2-(trimethylsilyl) ethoxy)methyl)-1H-imidazol-2-yl)-1-(phenylsulfonyl)-1H-indole (0.66 g, 1.08 mmol) and 30 mL of THF, and then the system was purged with argon for three times. Isopropylmagnesium chloride-lithium chloride complex (2 eq., 1.3 M, 2 mL, 2.16 mmol) was added dropwise at room temperature, and then the reaction was stirred at room temperature for 30 min. Compound 3,4,5-tri(methoxy-*d*₃)benzaldehyde (2 eq, 0.5 g, 2.16 mmol) was dissolved in 10 mL of anhydrous THF, and then added dropwise to the above reaction solution. The solution was stirred for 3 h, to which was added saturated NaCl solution to quench the reaction. The resultant solution was extracted with EA. The organic phase was rotatory evaporated to dry, and the residue was purified by column chromatography (EA/PE = 1/3), to provide compound (5-bromo-2-(1-(phenylsulfonyl)-1H-indol-3-yl)-1-(2-(trimethylsilyl)ethoxy)methyl)-1H-imidaz ol-4-yl)(3,4,5-tri(methoxy-d₃)phenyl)methanol (0.571 g, 0.77 mmol, 72%). LC/MS (ESI+) calcd for C₃₃H₂₉D₉Br₂N₃O₇SSi ([M+H]⁺) *m*/*z* 737.19, 739.19, found 737.2, 739.2.

### Step 6: Synthesis of (5-bromo-2-(1-(phenylsulfonyl)-1H-indol-3-yl)-1-(2-(trimethylsilyl) ethoxy)methyl)-1H-imidazol-4-yl)(3,4,5-tri(methoxy-d3)phenyl)ketone

To a 100 mL round-bottom flask, were added (5-bromo-2-(1-(phenylsulfonyl)-1H-indol-3-yl)-1-(2-(trimethylsilyl)ethoxy)methyl)-1H-imidazol-4-yl)(3,4,5-tri(methoxy-d₃)phenyl)meth anol (0.571 g, 0.77 mmol) and 30 mL of DCM, to which was added NaHCO₃ (4 eq., 0.31 g, 3.08 mmol) in an ice water bath, followed by slow addition of DMP (2 eq., 0.66 g, 1.56 mmol). The mixture was allowed to react for 1.5 h, and then saturated NaHCO₃ solution was slowly added to quench the reaction. The resultant solution was extracted with DCM. The organic phase was rotatory evaporated to dry, and the residue was purified by column chromatography (EA/PE = 1/4), to provide compound (5-bromo-2-(1-(phenylsulfonyl)-1H-indol-3-yl)-1-(2-(trimethylsilyl)ethoxy)methyl)-1H-imidazol-4-yl)(3,4,5-tri(methoxy-d3)phenyl)ketone (0.498 g, 0.68 mmol, 87%). LC/MS (ESI+) calcd for C₃₃H₂₇D₉Br₂N₃O₇SSi ([M+H]⁺) *m*/*z* 735.18, 737.18, found 735.2, 737.2.

### Steps 7 and 8: Synthesis of (2-(1H-indol-3-yl)-1H-imidazol-4-yl)(3,4,5-tri(methoxy-d₃) phenyl)ketone

To a 100 mL round-bottom flask, were added compound (5-bromo-2-(1-(phenylsulfonyl)-1H-indol-3-yl)-1-(2-(trimethylsilyl)ethoxy)methyl)-1H-imidazol-4-yl)(3,4,5-tri(methoxy-d3)ph enyl)ketone (0.498 g, 0.68 mmol), 5 mL of n-butanol, K₂CO₃ (4 eq., 0.376 g, 2.72 mmol), triphenylphosphine (0.4 eq., 0.071 g, 0.27 mmol), and palladium acetate ( 0.1 eq., 0.015 g, 0.07 mmol), and then the mixture was allowed to react for 5 h at 120 °C, to which was added saturated NaCl solution to quench the reaction. The resultant solution was extracted with EA. After evaporating n-butanol at high temperature, DCM/TFA (10 mL/5 mL) was directly added, and then the solution was stirred for 1.5 h. Then, saturated NaHCO₃ solution was slowly added to quench the reaction. The resultant solution was extracted with DCM. The organic phase was rotatory evaporated to dry, and the residue was purified by column chromatography, to provide compound HC-8174-01, (2-(1H-indol-3-yl)-1H-imidazol-4-yl)(3,4,5-tri(methoxy-d3)phenyl) ketone (0.25 g, 0.64 mmol, with a yield of 95% for two steps). LC/MS (ESI+) calcd for C₂₁H₁₀D₉N₃O₄ ([M+H]⁺) *m*/*z* 387.19, found 387.20. The target compound was a mixture of tautomeric isomers (3:2). ¹H NMR (400 MHz, DMSO-d₆) *δ* 13.07 (s, 1H), 11.61 (s, 1H), 8.45 (dd, *J=* 18.4, 7.8 Hz, 1H), 8.38 - 7.75 (m, 3H), 7.55 - 7.35 (m, 1H), 7.33 - 6.97 (m, 3H).

### Example 2: Synthesis of (2-(1H-indol-3-yl)-1H-imidazol-4-yl-5-d)(3,4,5-trimethoxyphenyl) ketone

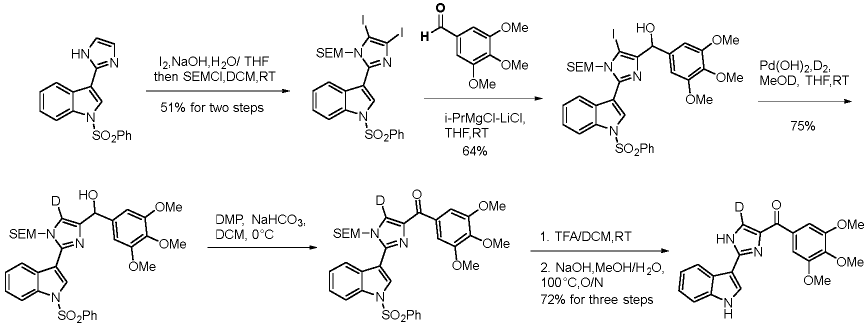

### Steps 1 and 2: Synthesis of 3-(4,5-diiodo-1-(2-(trimethylsilyl)ethoxy)methyl)-1H-imidazol-2-yl)-1-(phenylsulfonyl)-1H-indole

To a 100 mL round-bottom flask, were successively added 3-(1H-imidazol-2-yl)-1-(phenylsulfonyl)-1H-indole (2.65 g, 8.21 mmol), 50 mL of NaOH solution (2 M, 20 mL), and 10 mL of THF for solubilization, to which was slowly added iodine (2.5 eq., 5.2 g, 20.53 mmol), and then the mixture was stirred for 2 h. Saturated sodium thiosulfate solution was added to quench the reaction, and the resultant solution was extracted with EA for three times. The organic phase was rotatory evaporated to dry, followed by column chromatography. To the residue, were added 30 mL of DCM and DIPEA (1.1 mL), followed by slow addition of SEMCl (0.7 mL) in an ice water bath. The reaction was stirred for 2h, and then quenched by adding saturated NaHCO₃ solution. The resultant solution was extracted with DCM (4*30 mL), followed by purification over column chromatography (EA/PE = 1/5), to obtain compound 3-(4,5-diiodo-1-(2-(trimethylsilyl)ethoxy)methyl)-1H-imidazol-2-yl)-1-(phenylsulfonyl)-1H-in dole (2.19 g, 3.12 mmol, 51%). LC/MS (ESI+) calcd for C₂₃H₂₅I₂N₃O₃SSi ([M+H]⁺) *m*/*z* 705.95, found 706.1.

### Step 3. Synthesis of (5-iodo-2-(1-(phenylsulfonyl)-1H-indol-3-yl)-1-(2-(trimethylsilyl) ethoxy)methyl)-1H-imidazol-4-yl)(3,4,5-trimethoxyphenyl)methanol

To a 100 mL round-bottom flask, were added compound (4,5-diiodo-1-(2-(trimethylsilyl) ethoxy)methyl)-1H-imidazol-2-yl)-1-(phenylsulfonyl)-1H-indole (2.19 g, 3.12 mmol) and 30 mL of THF, and then the system was purged with argon for three times. Isopropylmagnesium chloride-lithium chloride complex (2 eq., 1.3 M, 2 mL, 2.16 mmol) was added dropwise at room temperature, and then the reaction was stirred at room temperature for 30 min. Compound 3,4,5-trimethoxybenzaldehyde (2 eq, 1.2 g, 6.2 mmol) was dissolved in 10 mL of anhydrous THF, and then added dropwise to the above reaction solution. The solution was stirred for 3 h, to which was added saturated NaCl solution to quench the reaction. The resultant solution was extracted with EA. The organic phase was rotatory evaporated to dry, and the residue was purified by column chromatography (EA/PE = 1/3), to provide compound (5-iodo-2-(1-(phenylsulfonyl)-1H-indol-3-yl)-1-(2-(trimethylsilyl)ethoxy)methyl)-1H-imidazol-4-yl)(3,4,5-trimethoxyphenyl)methanol (1.55 g, 1.98 mmol, 64%). LC/MS (ESI+) calcd for C₃₃H₃₈IN₃O₇SSi ([M+H]⁺) *m*/*z* 776.12, found 776.2.

### Step 4: Synthesis of (2-(1-(phenylsulfonyl)-1H-indol-3-yl)-1-(2-(trimethylsilyl)ethoxy) methyl)-1H-imidazol-4-yl-5-d)(3,4,5-trimethoxyphenyl)methanol

To a 100 mL round-bottom flask, was added (5-iodo-2-(1-(phenylsulfonyl)-1H-indol-3-yl)-1-(2-(trimethylsilyl)ethoxy)methyl)-1H-imidazol-4-yl)(3,4,5-trimethoxyphenyl)methanol (0.2 g, 0.26 mmol), which was dissolved in 5 mL of deuterated methanol, and 1 mL of anhydrous THF was added for solubilization. 0.2 g of palladium hydroxide was added, and then the system was purged with D₂ for three times. The mixture was allowed to react for 5 h under D₂ atmosphere. The reaction solution was directly subjected to suction filtration, and rotatory evaporated to dry. The residue was purified by column chromatography, to provide compound (2-(1-(phenylsulfonyl)-1H-indol-3-yl)-1-(2-(trimethylsilyl)ethoxy)methyl)-1H-imidazol-4-yl-5-*d*)(3,4,5-trimethoxyphenyl)methanol (0.128 g, 0.194 mmol, 75%). LC/MS (ESI+) calcd for C₃₃H₃₈DN₃O₇SSi ([M+H]⁺) *m*/*z* 651.23, found 651.20.

### Steps 5, 6, and 7: Synthesis of (2-(1H-indol-3-yl)-1H-imidazol-4-yl-5-d) (3,4,5-trimethoxyphenyl)ketone

To a 100 mL round-bottom flask, were added compound (1-(phenylsulfonyl)-1H-indol-3-yl)-1-(2-(trimethylsilyl)ethoxy)methyl)-1H-imidazol-4-yl-5-*d*)(3,4,5-trimethoxyphenyl)metha nol (0.128 g, 0.197 mmol) and 30 mL of DCM, to which was added NaHCO₃ (4 eq., 0.068 g, 0.78 mmol) in an ice water bath, followed by slow addition of DMP (2 eq., 0.25 g, 0.39 mmol). The mixture was allowed to react for 1.5 h, and then saturated NaHCO₃ solution was slowly added to quench the reaction. The resultant solution was extracted with DCM, to obtain the crude product (2-(1-(phenylsulfonyl)-1H-indol-3-yl)-1-(2-(trimethylsilyl)ethoxy)methyl)-1H-imidazol-4-yl-5-d)(3,4,5-trimethoxyphenyl)ketone, which was directly used in the next step.

To a 100 mL round-bottom flask, was added the crude product (2-(1-(phenylsulfonyl)-1H-indol-3-yl)-1-(2-(trimethylsilyl)ethoxy)methyl)-1H-imidazol-4-yl-5-d)(3,4,5-trimethoxyphe nyl)ketone, to which was added DCM/TFA (10 mL/5 mL) at room temperature, and then the solution was stirred for 1.5 h. Then, saturated NaHCO₃ solution was slowly added to quench the reaction. The resultant solution was extracted with DCM. The organic phase was rotatory evaporated to dry, and to the residue, were added NaOH (20 eq, 0.16 g, 3.88 mmol) and methanol/water = 5 mL/20 mL. The solution was stirred overnight at 100 °C, and then extracted with EA. The organic phase was rotatory evaporated to dry, and the residue was purified by column chromatography, to provide compound **HC-8550-01,** (2-(1H-indol-3-yl)-1H-imidazol-4-yl-5-d)(3,4,5-trimethoxyphenyl)ketone (0.054 g, 0.140 mmol, with a yield of 72% for three steps). LC/MS (ESI+) calcd for C₂₁H₁₈DN₃O₄ ([M+H]⁺) *m*/*z* 379.14, found 379.20. The target compound was a mixture of tautomeric isomers (3:2). ¹H NMR (400 MHz, DMSO-d₆) *δ* 13.07 (s, 1H), 11.61 (s, 1H), 8.58 - 7.83 (m, 3H), 7.56 - 7.37 (m, 1H), 7.26 - 7.06 (m, 3H)., 3.91 (s, 6H), 3.78 (s, 3H).

### Example 3: Synthesis of (2-(1H-indol-3-yl)-1H-imidazol-4-yl-5-d)(3,4,5-tri(methoxy-d₃) phenyl)ketone

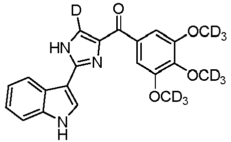

The target compound was synthesized by the methods similar to those in Examples 1 and 2. LC/MS (ESI+) calcd for C₂₁H₉D₁₀N₃O₄ ([M+H]⁺) *m*/*z* 379.14, found 379.20. The target compound was a mixture of tautomeric isomers (3:2). ¹H NMR (400 MHz, DMSO) *δ* 13.1 (s, 1H), 11.6 (s, 1H), 8.43 (d, *J =* 7.7 Hz, 1H), 7.53 - 7.03 (m, 5H).

### Example 4: Synthesis of (2-(1H-indol-3-yl)-1H-imidazol-4-yl-5-d)(3,5-dimethoxy-4-(methoxy-d₃)phenyl)ketone

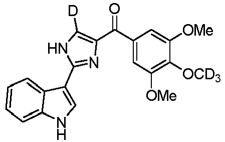

The target compound was synthesized by the methods similar to those in Examples 1 and 2. LC/MS (ESI+) calcd for C₂₁H₁₅D₄N₃O₄ ([M+H]⁺) *m*/*z* 379.14, found 379.20. The target compound was a mixture of tautomeric isomers (3:2). ¹H NMR (400 MHz, DMSO) *δ* 13.0 (d, *J* = 53.5 Hz, 1H), 11.6 (d, *J =* 53.6 Hz, 1H), 8.44 (dd, *J =* 18.6, 7.7 Hz, 1H), 8.37 - 7.91 (m, 2H), 7.53 - 7.39 (m, 1H), 7.24 - 7.07 (m, 3H), 3.91 (s, 6H).

### Example 5: Synthesis of (2-(1H-indol-3-yl)-1H-imidazol-4-yl-5-d)(3,4-dimethoxy-5-(methoxy-d₃)phenyl)ketone

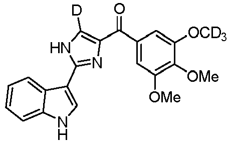

The target compound was synthesized by the methods similar to those in Examples 1 and 2. LC/MS (ESI+) calcd for C₂₁H₁₅D₄N₃O₄ ([M+H]⁺) *m*/*z* 379.14, found 379.20. The target compound was a mixture of tautomeric isomers (3:2). ¹H NMR (400 MHz, DMSO) *δ* 13.0 (s, 1H), 11.54 (s, 1H), 8.45 (dd, *J =* 18.0, 7.9 Hz, 1H), 8.39 - 7.89 (m, 2H), 7.56 - 7.37 (m, 1H), 7.26 - 7.07 (m, 3H), 3.91 (s, 3H), 3.78 (s, 3H).

### Example 6: Synthesis of (2-(1H-indol-3-yl)-1H-imidazol-4-yl-5-d)(3-methoxy-4,5-di(methoxy-d₃)phenyl)ketone

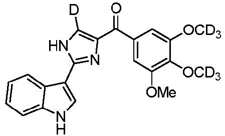

The target compound was synthesized by the methods similar to those in Examples 1 and 2. LC/MS (ESI+) calcd for C₂₁H₁₂D₇N₃O₄ ([M+H]⁺) *m*/*z* 385.18, found 385.20. ¹H NMR (400 MHz, DMSO) *δ* 13.1 (s, 1H), 11.6 (s, 1H), 8.43 (d, *J =* 7.7 Hz, 1H), 8.46 - 7.61 (m, 2H), 7.47 (d, *J= 8.0* Hz, 1H), 7.23 - 7.10 (m, 3H), 3.90 (s, 3H).

### Example 7: Synthesis of (2-(1H-indol-3-yl)-1H-imidazol-4-yl-5-d)(4-methoxy-3,5-di(methoxy-d₃)phenyl)ketone

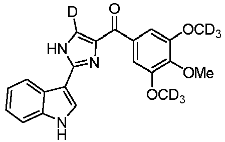

The target compound was synthesized by the methods similar to those in Examples 1 and 2. LC/MS (ESI+) calcd for C₂₁H₁₂D₇N₃O₄ ([M+H]⁺) *m*/*z* 385.18, found 385.20. The target compound was a mixture of tautomeric isomers (3:2). ¹H NMR (400 MHz, DMSO-d₆) *δ* 13.07 (s, 1H), 11.61 (s, 1H), 8.43 (dt, *J =* 26.9, 13.6 Hz, 1H), 8.38 - 7.94 (m, 1H), 7.93 (s, 1H), 7.53 - 7.38 (m, 1H), 7.30 - 7.03 (m, 3H), 3.78 (s, 3H).

The beneficial effects of the present invention were demonstrated by reference to the Experimental examples in the following.

### Experimental example 1: Metabolic stability experiment of the compound according to the present invention in human hepatocyte microsomes

Step 1. the mother solution was prepared with phosphate buffer (200 mM), high-purity water, and MgCl₂ solution (50 mM), wherein the concentration of phosphate was 100 mM, while the concentration of MgCl₂ was 5 mM.

Step 2: Two experiments were carried out as follows:
A) Addition of reduced coenzyme II (NADPH): 10 µL of human hepatocyte microsomes (20 mg/mL) and 40 µL of NADPH (10 mM) were added to the incubation experiment. The final concentrations of liver microsomes and NADPH were 0.5 mg/mL and 1 mM, respectively.
B) No addition of NADPH: 10 µL of human hepatocyte microsomes (20 mg/mL) and 40 µL of high-purity water were added to the incubation experiment. The final concentration of liver microsomes was 0.5 mg/mL.

Step 3: A positive control or test compound was added, and then the reaction started. The final concentration of the test compound was 2 µM.

Step 4: 50 µL of solution was collected from each reaction solution at the time points 0 min, 15 min, 30 min, 45 min, and 60 min. To the reaction solution, were add 4× volume of acetonitrile and IS (100 nM alprazolam, 200 nM labetalol, 200 nM caffeine, and 2 µM ketoprofen). The sample was centrifuged for 40 min. 100 µL of the supernatant was collected, to which was added 100 µL of high-purity water for LC-MS/MS analysis. The peak area was determined from the extracted ion chromatogram. The natural logarithm of the remaining percentage of the parent drug and the incubation time were plotted to obtain the slope (*k*) by linear regression. The *in vitro* half-life (*in vitro* t_{1/2}) was determined by the slope value.

The experimental results of the metabolic stability for the compound of the present invention in human liver microsomes are shown in Table 1:

**Table 1: The half-life of compounds according the present invention in human liver microsomes.**

| | Sabizabuli n | Example 1 | Example 2 | Exampl e 3 | Exampl e 4 | Exampl e 5 | Exampl e 6 | Exampl e 7 |
|---|---|---|---|---|---|---|---|---|
| T_{1/2} (min) | 88 | 100 | 141 | 155 | 98 | 112 | 112 | 71 |

As shown in Table 1, the compound of the present invention showed a longer half-life period than Sabizabulin in human liver microsome experiments, indicating that the compound of the present invention had better human metabolic stability than Sabizabulin and was expected to have better human pharmacokinetics.

### Experimental example 2: Pharmacokinetics of the compound according to the present invention in mice

1) Experimental materials and instruments:
   LC-20AD high performance liquid chromatography system (SHIMADZU, Japan)
   API4000 triple quadrupole mass spectrometer (Applied Biosystem, USA)
   PhenixWinnolin Pharmacokinetic software (Version 6.3, Certara, USA)
   High speed freezing centrifuge (Thermo Fisher Scientific)
   Analytical balance (Sartorius, SECURA225D-1CN)
   ICR mice (Chengdu Dossy Experimental Animals Co., Ltd)
2) Experimental methods and results

A suitable amount of drugs was accurately weighed respectively, and then according to the final volume of the preparation, 5% DMSO, 15% HS-15, and 80% D5W were successively added at their proportions. The solution was mixed well with the aid of ultrasound and vortex. Thus, the preparation with a drug concentration of 0.3 mg/ml (i.g.) was separately obtained.

18 healthy adult ICR mice were divided into two groups (9 for the i.v. group and 9 for the i.g. group). After fasting overnight (free drinking water), mice were administered via tail vein or gavage, respectively. The dosage of oral administration was 3 mg/kg. Using satellite blood culture system, 3 animals were collected at each time point, and 0.1 ml of blood was collected at different time points and anticoagulated with EDTA-K2. The plasma was separated by centrifugation at 4 °C for 5 min and stored at -80 °C for testing. For i.v., the sampling time points after administration were 5 min, 15 min, 0.5 h, 1 h, 2 h, 4 h, 8 h, 12 h, and 24 h; while, for i.g., the sampling time points were before administration (0 h), and 0.5 h, 1 h, 2 h, 4 h, 6 h, 8 h, 12 h, and 24 h after administration. The drug concentration in plasma was determined by LC/MS/MS. The pharmacokinetic parameters in mice are shown in Table 2.

**Table 2. Pharmacokinetic parameters of the compound according to the present invention in mice (oral administration, 3 mg/kg).**

| Compound | Peak concentration Cₘₐₓ (ng/mL) | Exposure dose AUC_{inf} (ng*h/mL) |
|---|---|---|
| Sabizabulin | 507 | 1364 |
| Example 1 | 556 | 1371 |
| Example 2 | 906 | 2748 |

As shown in Table 2, the compounds of the present invention showed higher exposure levels in mice.

### Experimental example 3: Pharmacokinetics of the compound according to the present invention in rats

1) Experimental materials and instruments:
   LC-20AD high performance liquid chromatography system (SHIMADZU, Japan)
   API4000 triple quadrupole mass spectrometer (Applied Biosystem, USA)
   PhenixWinnolin Pharmacokinetic software (Version 6.3, Certara, USA)
   High speed freezing centrifuge (Thermo Fisher Scientific)
   ICR mice (Chengdu Dossy Experimental Animals Co., Ltd)
2) Experimental methods and results

A suitable amount of drugs was accurately weighed (equivalent to 3 mg of prototype drug), and then according to the final volume of the preparation, 5% DMSO, 15% HS-15, and 80% D5W were successively added at their proportions. The solution was mixed well with the aid of ultrasound and vortex.

Three healthy adult male SD rats (180-250 g) were fasted overnight (free access to water) and administered by gavage at a volume of 5 ml/kg. 0.1 ml of blood was collected from the post-orbital venous plexus before administration as well as 0.5 h, 1 h, 2 h, 4 h, 6 h, 8 h, 12 h, and 24 h after administration. The plasma was separated by centrifugation at 4 °C for 5 min and stored at -20 °C for testing. Then, the concentration of the test compound in the plasma was determined by LC/MS/MS.

**Table 3. Pharmacokinetic parameters of the compound according to the present invention in rats (oral administration, 3 mg/kg).**

| Compound | Peak concentration Cₘₐₓ (ng/mL) | Exposure dose AUC_{inf} (ng*h/mL) |
|---|---|---|
| Sabizabulin | 612 | 1582 |
| Example 2 | 726 | 2918 |
| Example 3 | 170 | 1974 |

As shown in Table 3, the compounds of the present invention showed higher exposure levels in rats.

In summary, the deuterated Sabizabulin provided in the present invention had better human metabolic stability and animal pharmacokinetics compared with Sabizabulin, was expected to have better human pharmacokinetics, and correspondingly showed excellent application prospects.

## Claims

1. A compound represented by formula (I) or an optical isomer, a pharmaceutically acceptable salt, a hydrate, or a solvate thereof: 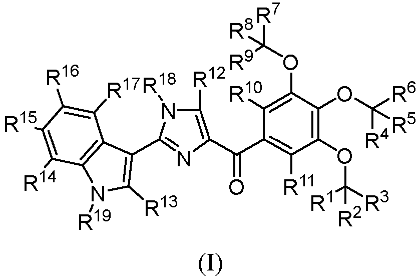 wherein R¹-R¹⁷ are each independently selected from hydrogen and deuterium, R¹⁸ and R¹⁹ are hydrogen, and R¹-R¹⁹ are not hydrogen at the same time.

2. The compound according to claim 1 or an optical isomer, a pharmaceutically acceptable salt, a hydrate, or a solvate thereof, **characterized in that** the compound has a structure as represented by formula (II):

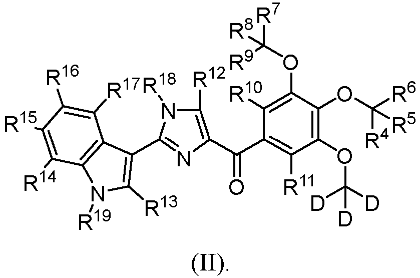

3. The compound according to claim 1 or an optical isomer, a pharmaceutically acceptable salt, a hydrate, or a solvate thereof, **characterized in that** the compound has a structure as represented by formula (III):

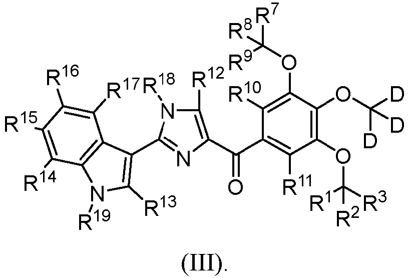

4. The compound according to claim 2 or 3 or an optical isomer, a pharmaceutically acceptable salt, a hydrate, or a solvate thereof, **characterized in that** the compound has a structure as represented by formula (IV):

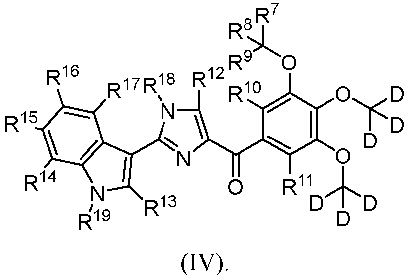

5. The compound according to claim 4 or an optical isomer, a pharmaceutically acceptable salt, a hydrate, or a solvate thereof, **characterized in that** the compound has a structure as represented by formula (V):

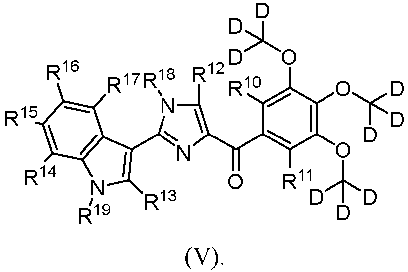

6. The compound according to claim 1 or an optical isomer, a pharmaceutically acceptable salt, a hydrate, or a solvate thereof, **characterized in that** the compound has a structure as represented by formula (VI):

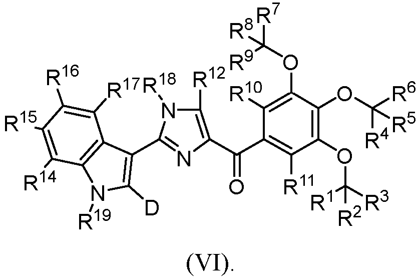

7. The compound according to claim 1 or an optical isomer, a pharmaceutically acceptable salt, a hydrate, or a solvate thereof, **characterized in that** the compound has a structure as represented by formula (VII):

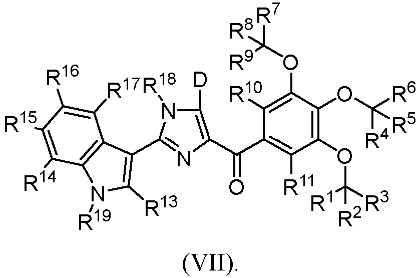

8. The compound according to claim 1 or an optical isomer, a pharmaceutically acceptable salt, a hydrate, or a solvate thereof, **characterized in that** the compound has a structure as represented by formula (VIII):

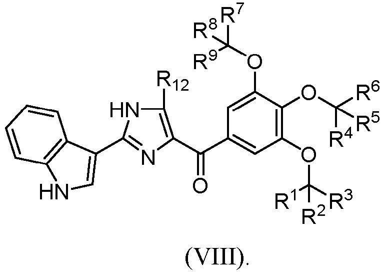

9. The compound according to claim 8 or an optical isomer, a pharmaceutically acceptable salt, a hydrate, or a solvate thereof, **characterized in that** R₁, R₂, and R₃ are all hydrogen at the same time or are all deuterium at the same time; R₄, R₅, and R₆ are all hydrogen at the same time or are all deuterium at the same time; R₇, R₈, and R₉ are all hydrogen at the same time or are all deuterium at the same time.

10. The compound according to any one of claims 1 to 9 or an optical isomer, a pharmaceutically acceptable salt, a hydrate, or a solvate thereof, **characterized in that** the compound is selected from the group consisting of:

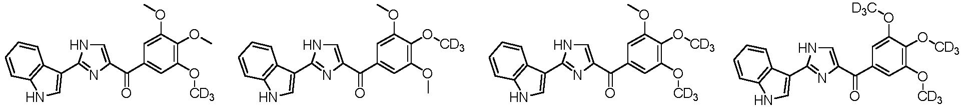

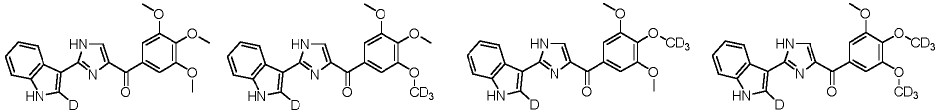

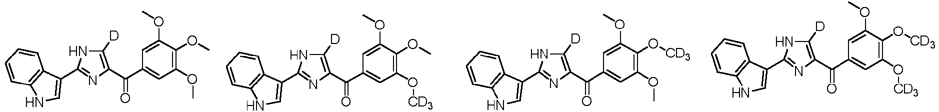

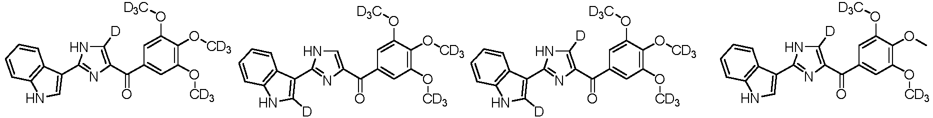

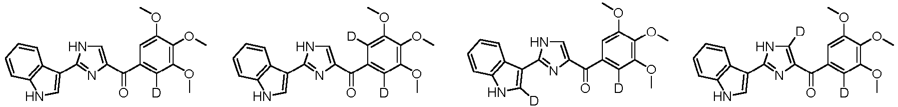

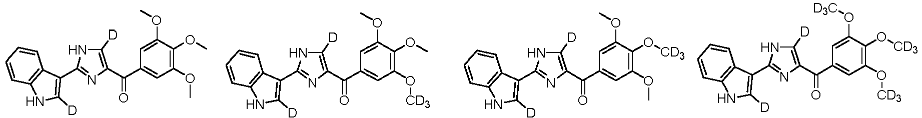

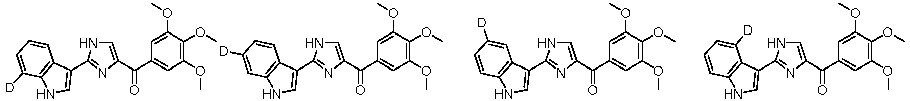

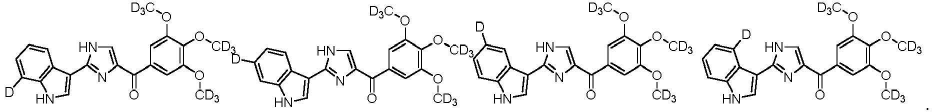

11. The compound according to any one of claims 1 to 10 or an optical isomer, a pharmaceutically acceptable salt, a hydrate, or a solvate thereof, **characterized in that** the pharmaceutically acceptable salt includes phosphate, D-camphorsulfonate, hydrochloride, hydrobromate, hydrofluorate, sulfate, nitrate, formate, acetate, propionate, oxalate, malonate, succinate, fumarate, maleate, lactate, malate, tartrate, citrate, picrate, methanesulfonate, phenylmethanesulfonate, benzenesulfonate, aspartate or glutamate of the compound.

12. The compound according to any one of claims 1 to 11 or an optical isomer, a pharmaceutically acceptable salt, a hydrate, or a solvate thereof for use in the manufacture of medicaments for the treatment of cancer, COVID-19 infection, influenza virus infection, syncytial virus (SV) infection and/or acute respiratory distress syndrome (ARDS).

13. The use according to claim 12, **characterized in that** the cancer includes melanoma, prostate cancer or breast cancer.

14. The compound according to any one of claims 1 to 11 or an optical isomer, a pharmaceutically acceptable salt, a hydrate, or a solvate thereof for use in the manufacture of tubulin inhibitors.

15. A medicament, **characterized in that** it is a preparation formed by the compound according to any one of claims 1 to 11 or an optical isomer, a pharmaceutically acceptable salt, a hydrate, or a solvate thereof, as the active ingredient, in combination with pharmaceutically acceptable excipients.
